# EUROPÄISCHE PATENTSCHRIFT

(11) **EP 0 375 901 B1**
(45) Veröffentlichungstag und Bekanntmachung des Hinweises auf die Patenterteilung: **08.02.1995**
(21) Anmeldenummer: 89120923.1
(22) Anmeldetag: 11.11.1989
(51) Int. Cl.: A61B 17/12

(54) **Abschnürvorrichtung für Körperteile**
Ligaturing device for parts of the human body
Dispositif de ligature pour des parties du corps humain

(30) Priorität: 26.11.1988 DE 3840007
(43) Veröffentlichungstag der Anmeldung: 04.07.1990
(73) Patentinhaber: PRÄMETA Gesellschaft für Präzisionsmetall- und Kunststofferzeugnisse mbH & Co. KG, 51107 Köln (DE)
(72) Erfinder: Wehking, Wolfgang, D-5000 Köln 90 (DE)
(74) Vertreter: Dallmeyer, Georg, Dipl.-Ing.

(56) Entgegenhaltungen:
- EP-A- 0 196 646
- DE-A- 2 824 037
- FR-A- 2 321 264

## Beschreibung

Die Erfindung bezieht sich auf eine Abschnürvorrichtung für Körperteile mit einem Schloßgehäuse, einem in dem Schloßgehäuse schwenkbar gelagerten Klemmhebel für das durch das Schloßgehäuse hindurchgeführte Band und einem das andere Bandende tragenden und in das Schloßgehäuse einrastbaren Schuh, wobei das Schloßgehäuse an dem der Bandschlinge zugekehrten Ende freistehende Seitenwangen aufweist, die den Klemmhebel seitlich begrenzen.

Eine solche Abschnürvorrichtung, z.B. nach DE-C 25 41 433, hat sich in hervorragendem Maß bewährt. Das Öffnen der Bandschlinge durch Ausrasten des das Bandende aufnehmenden Schuhs von dem Klemmhebel kann leicht mit einer Hand, und zwar mittels des Daumens der Hand, durchgeführt werden, so daß die Bandschlinge durch Abspringen des einrastbaren Schuhes von dem Klemmhebel gelöst werden kann. Man erzielt eine einfache und sichere Öffnung der Abschnürvorrichtung, ohne daß es notwendig ist, die Bandschlinge bei Abnehmen der Abschnürvorrichtung längs des Körperteils zu führen.

Bei der bekannten Lösung befinden sich die der Gurtschlaufe zugekehrten Enden der Seitenwangen des Schloßgehäuses und des Klemmhebels in so geringem, gemeinsamen Abstand zur Oberfläche des umspannten Körperteils, daß es besonders bei schlaffen, auf dem Fettgewebe des umspannten Körperteils leicht verschiebbaren Häuten gelegentlich vorkommen kann, daß die Haut beim Anziehen des Gurtes in die Öffnung an der Abschnürvorrichtung zwischen dem einen Wandende und dem anderen in Form einer Falte eindringt und von der unter Druck anliegenden Oberfläche der durchlaufenden Gurtschlinge in den horizontalen Spalt zwischen Klemmhebel und Schloßgehäuse oder sogar in den Raum unterhalb der Verriegelung des das Bandende aufnehmenden Schuhs hineingezogen wird, was zu Klemmungen an der Haut führen kann. Die betreffende Person verspürt ein mehr oder weniger wirksames Kneifen der Haut an der Stelle, an der die Stirnfläche der Abschnürvorrichtung dem Körperteil zugekehrt ist. Eine Lockerung der Gurtspannung durch Niederdrücken des Klemmhebels ist wegen der in den Spalt zwischen Klemmhebel und Schloßgehäuse eingedrungenen Haut nicht mehr möglich. In einzelnen Fällen kann es beim Versuch der Lösung der Bandspannung durch Niederdrücken des Bandendenschuhs zu weiterer Schmerzzunahme wegen des entstehenden Druckes auf die in das Fenster des Klemmhebels eingezogenen, unmittelbar unter dem Schuh liegenden Hautpartien kommen.

Eine weitere gattungsgemäße Abschnürvorrichtung für Körperteile ist aus der DE-A-28 24 037 bekannt. Das Gehäuse dieser Abschnürvorrichtung steht gegenüber dem Klemmhebel sowohl hinsichtlich der Seitenwangen als auch hinsichtlich des Bodens vor, wobei die der Bandschlinge zugewandten Stirnflächen der Seitenwände relativ zu der Vorderkante des Bodens vorstehen.

Der Klemmhebel ist mit einem Befestigungsmittel für das eine Bandende der Bandschlaufe verbunden, wobei das starr mit dem Klemmhebel verbundene Befestigungsmittel als Verlängerung des Klemmhebels wirkt. Der Abstand der Bandenden der Bandschlaufe an dem Ende des Schloßgehäuses, wo die beiden Bandenden eingeführt sind, ist so gering, daß das eingangs geschilderte Problem des Einziehens einer Hautfalte auch bei dieser Abschnürvorrichtung besteht.

Aufgabe der Erfindung ist es, eine Abschnürvorrichtung der vorstehend genannten Art zu schaffen, bei der die Gefahr eines Klemmens der Haut an der Anlegestelle der Abschnürvorrichtung mit Sicherheit beseitigt ist.

Zur Lösung dieser Aufgabe dienen die Merkmale des Anspruchs 1.

Durch eine solche Ausbildung der Abschnürvorrichtung hat eine durch den Gurt eventuell aufgeschobene Hautfalte genügend Raum zwischen den beiden Gurtenden, der durch die Seitenwangen begrenzt wird, um sich zu entspannen und damit wieder von der Gurtinnenfläche zu lösen. Die Stirnfläche des Klemmhebels liegt so weit zurück, daß die Haut auch bei schwammiger Art, keine Gelegenheit findet, sich in den Spalt zwischen Klemmhebel und Schloßgehäuse einlegen zu können. Die Haut wird weder bei Betätigung des Klemmhebels noch des ausrastbaren Schuhs in Mitleidenschaft gezogen. Die Seitenwangen und der Boden des Schloßgehäuses sind zu dem Klemmhebel vorstehend angeordnet sind.

Gemäß einem weiteren Merkmal der Erfindung ist die Anordnung so getroffen, daß auch die zur Bandschlinge gerichtete Stirnkante des Gehäusebodens zu der Stirnfläche des Klemmhebels vorragt. Dadurch ergibt sich eine Vergrößerung der Anlagefläche der Haut an der Stirnseite der Abschnürvorrichtung. Der spezifische Flächendruck wird vermindert.

Hierbei ist es zweckmäßig, daß die Stirnkante der Seitenwangen und des Gehäusebodens - im Querschnitt gesehen - gerundet ausgebildet ist. Weiterhin kann die Stirnkante des Gehäusebodens nach unten, entsprechend dem Einlaufradius des Gurtes, abgebogen verlaufen.

Die Erfindung wird anhand eines in der Zeichnung dargestellten Ausführungsbeispieles näher erläutert:

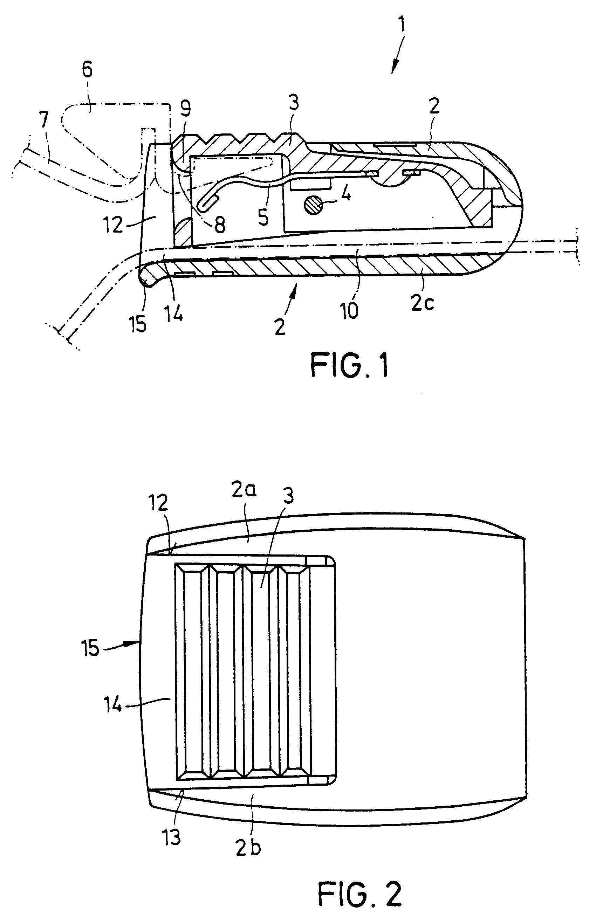

- Fig. 1: zeigt eine Ausführungsform der Abschnürvorrichtung der Erfindung im Längsschnitt und im Schema.
- Fig. 2: stellt eine Draufsicht auf die Abschnürvorrichtung der Fig. 1 dar.

Die Abschnürvorrichtung 1 weist ein Schloßgehäuse 2 auf, in dessen Hohlraum ein Klemmhebel 3 um eine Achse 4 schwenkbar gelagert ist. Der Klemmhebel 3 steht vorteilhaft unter der Wirkung einer Feder 5, die zugleich dazu dient, daß ein Schuh 6, an dem das eine Bandende 7 befestigt ist, in der Raststellung federnd festgehalten wird. Hierzu besitzt der Schuh 6 eine Nut 8, die im eingerasteten Zustand hinter einer Querrippe 9 des Klemmhebels 3 greift. Das andere Bandende 10 ist durch das Schloßgehäuse 2 hindurchgeführt und kann durch den Klemmhebel 3 festgeklemmt gehalten werden.

Die Seitenwangen 2a und 2b sowie der Boden 2c des Schloßgehäuses 2 sind gegenüber der Stirnfläche des Klemmhebels 3 bei 12, 13, 14 vorstehend angeordnet. Es genügt hierbei ein Bereich von 3 bis 6 mm und darüber. Ferner sind die Stirnflächen der Seitenwangen und des Bodens 2c - im Querschnitt gesehen - gerundet ausgebildet.

Zur Verkleinerung des spezifischen Anlagedruckes kann die vorstehende Stirnkante 15 des Gehäusebodens 2c etwas nach unten abgebogen verlaufen.

Durch die zurückspringende Anordnung der Stirnfläche des Klemmhebels 3 verbleibt selbst beim strammen Anziehen der Abschnürvorrichtung noch ein genügender Raum, um eine Beeinträchtigung auch schlaffer Haut auch unter diesen Bedingungen nicht mehr zuzulassen.

## Patentansprüche

1. Abschnürvorrichtung für Körperteile mit einem mit einem Bandende (10) durch ein Schloßgehäuse (2) geführten Band, einem in dem Schloßgehäuse (2) zwischen Seitenwangen (2a, 2b) schwenkbar gelagerten Klemmhebel (3), der das in einem Spalt zwischen dem Klemmhebel (3) und einem dem Klemmhebel (3) gegenüberliegendem Boden (2c) des Schloßgehäuses hindurchgeführte Bandende (10) einklemmt und mit einem das andere Bandende (7) aufnehmenden und unter Bildung einer Bandschlinge mit dem Klemmhebel (3) verriegelbaren Schuh (6), wobei das Schloßgehäuse (2) an dem Ende, wo die beiden Bandenden (7,10) eingeführt sind, frei- und vorstehende Seitenwangen (2a,2b) aufweist,
**dadurch gekennzeichnet,**
daß zwischen dem Schuh im verriegelten Zustand (6) und dem gegenüber dem Klemmhebel (3) vorstehenden Boden (2c) des Schloßgehäuses (2) ein solcher Abstand eingehalten ist, daß ein Raum zwischen den beiden Bandenden (7,10) verbleibt, in dem sich eine ggf. von dem Bandende (10) eingezogene Hautfalte entspannen und sich von der Bandinnenseite lösen kann.

2. Abschnürvorrichtung nach Anspruch 1, dadurch gekennzeichnet, daß die zur Bandschlinge gerichtete Stirnkante des Gehäusebodens (2c) zu den Stirnflächen der Seitenwangen (2a,2b) vorragt.

3. Abschnürvorrichtung nach Anspruch 1 oder 2, dadurch gekennzeichnet, daß sowohl die Stirnkante des Gehäusebodens (2c) als auch die Stirnkanten der Seitenwangen (2a,2b) - im Querschnitt gesehen - unabhängig voneinander gerundet ausgebildet sind.

4. Abschnürvorrichtung nach einem der Ansprüche 1 bis 3, dadurch gekennzeichnet, daß die Stirnkante (15) des Gehäusebodens (2c) nach unten abgebogen verläuft.

## Claims

1. A tourniquet device for extremities comprising a strap, one end (10) of which is passed through a lock casing (2), a clamping lever (3), pivotally supported within said lock casing (2) between lateral cheeks (2a, 2b), which clamping lever clamps said strap end (10) passed through a gap between said clamping lever (3) and a bottom (2c) of said lock casing facing said clamping lever (3), and a shoe (6) receiving the other end (7) of said strap and, forming a strap loop, being engageable with said clamping lever (3), said lock casing (2) having self-supported and projecting lateral cheeks (2a,2b) at the end where said two strap ends (7,10) are introduced,
**characterised in that**
such a distance is kept between the shoe (6) in its engaged state and the bottom (2c) of the lock casing (2) projecting with respect to the clamping lever (3) that a space remains between the two strap ends (7,10) in which a skin fold possibly drawn in by the strap end (10) can relax and disengage from the inner side of the strap.

2. The tourniquet device according to claim 1, characterised in that the front edge of said casing bottom (2c) facing towards said strap loop projects with respect to the front edges of said lateral cheeks (2a, 2b).

3. The tourniquet device according to claim 1 or 2, characterised in that - seen in cross section - both the front edge of said casing bottom (2c) and the front edges of said lateral cheeks (2a, 2b) are each independently rounded.

4. The tourniquet device according to one of claims 1 to 3, characterised in that said front edge (15) of said casing bottom (2c) extends in a downward bend.

## Revendications

1. Dispositif de ligature pour des parties du corps humain, avec une courroie passée par une extrémité de courroie (10) dans un boîtier de fermoir (2), un levier de serrage (3), monté de manière pivotante dans le boîtier de fermoir (2), entre des joues latérales (2a, 2b), qui serre l'extrémité de courroie (10) passée dans une fente entre le levier de serrage (3) et un fond (2c) du boîtier de fermoir situé en face du levier de serrage (3), et avec un sabot (6) recevant l'autre extrémité de courroie (7) et verrouillable avec le levier de serrage (3) en formant une boucle de courroie, le boîtier de fermoir (2) présentant, à l'extrémité où sont introduites les deux extrémités de courroie (7, 10), des joues latérales (2a, 2b) libres et en saillie, caractérisé en ce qu'entre le sabot (6) à l'état verrouillé et le fond (2c) du boîtier de fermoir (2), en saillie par rapport au levier de serrage (3), est maintenue une distance telle qu'il reste un espace entre les deux extrémités de courroie (7, 10) dans lequel un plissement de la peau éventuellement introduit par l'extrémité de courroie (10) peut se détendre et se détacher de la face intérieure de la courroie.

2. Dispositif de ligature suivant la revendication 1, caractérisé en ce que le bord frontal, orienté vers la boucle de courroie, du fond de boîtier (2c) est en saillie par rapport aux faces frontales des joues latérales (2a, 2b).

3. Dispositif de ligature suivant la revendication 1 ou 2, caractérisé en ce que tant le bord frontal du fond de boîtier (2c) que les bords frontaux des joues latérales (2a, 2b) - vus en section - se présentent sous forme arrondie, indépendamment l'un de l'autre.

4. Dispositif de ligature suivant l'une des revendications 1 à 3, caractérisé en ce que le bord frontal (15) du fond de boîtier (2c) s'étend arqué vers le bas.
